# EUROPEAN PATENT SPECIFICATION

(11) **EP 3 589 341 B1**
(45) Date of publication and mention of the grant of the patent: **04.06.2025**
(21) Application number: 16833990.1
(22) Date of filing: 06.08.2016
(51) Int. Cl.: A61M 11/02

(54) **REMOTE CONTROLLED MEDICINE DELIVERY SYSTEM**
FERNGESTEUERTES MEDIKAMENTENABGABESYSTEM
SYSTÈME D'ADMINISTRATION DE MÉDICAMENT COMMANDÉ À DISTANCE

(30) Priority: 06.08.2015 US 201562201815 P; 05.08.2016 US 201615230143
(43) Date of publication of application: 08.01.2020
(73) Proprietor: Bemis Manufacturing Company, Falls WI 53085-0901 (US)
(72) Inventor: SCHWAB, Brian, East Chatham, NY 12060 (US)
(74) Representative: Clark, Jonathan Lister
(86) International application number: PCT/US2016/045932
(87) International publication number: WO 2017/024294

(56) References cited:
- EP-A1- 2 138 640
- WO-A1-2013/020240
- US-A- 2 875 450
- US-A- 4 422 189
- US-A- 5 101 520
- US-A- 6 009 570
- US-A1- 2009 313 752
- US-A1- 2009 313 752
- US-A1- 2011 203 044
- US-A1- 2012 005 817
- US-A1- 2013 180 041
- US-A1- 2014 068 862
- US-A1- 2015 337 525
- US-B1- 7 120 946

## Description

### CROSS-REFERENCE TO RELATED APPLICATIONS

This application claims benefit of United States provisional patent application serial number 62/201,815, filed August 6, 2015, and United States patent application serial number 15/230,140, filed August 5, 2016.

### BACKGROUND OF THE INVENTION

Embodiments of the present disclosure generally relate to the delivery and application of medicine. More specifically, aspects of the present disclosure provide a solution for delivering and/or applying medicine to a region of the body (e.g., perianal region) that may difficult for the user to access. More specifically, aspects of the present disclosure provide a method and a wash system with nozzles for washing and applying medication, to a region of a human body (e.g., skin, genital or anal area, intimate parts, perianal region) and cleaning thereof.

### Description of the Related Art

Many maladies that are treated today existed in antiquity. Bodily sores, lesions, rashes, hemorrhoids, and the like have a long history whose treatment often included the application of a medicinal substance of one kind or another to the source of irritation. Although medicine has advanced greatly in recent times, existing medicinal delivery methods still usually involve the same principles of application as those used in antiquity; the patient must hold the medicinal applicator by hand, and then contort their body to enable the medicine (be it in the form of aerosol spray, pump mist, cream, *etc.*) to reach the affected area. This often results in an uncomfortable, imprecise method of delivery when self-administered by the user. Alternatively, having a third-party administer the medicine can be embarrassing. Thus, there is a need to provide a remote-controlled guidance system for delivery of a medicine to a region of a body.

On the other hand, bidets and other modern toilet seat systems have been used to spray water and clean private parts of a user using a toilet. The bidet systems are used for washing the genital and anal areas using cleaning water of appropriate temperature sprayed from the center of the bidet system, instead of a toilet paper after relief stool or urination. Originally being developed for washing the pubic area for females, bidet systems have now been popular among people of all ages and both sexes because it is known to be more hygienic to wash the intimate parts and anus with water instead of paper after relief. In addition, cleansing the pubic/anal regions with water may help to avoid infection and prevent hemorrhoids and other anal disease. Furthermore, it is very effective for women with gynecology diseases. It is also very useful for the elderly or obese people to relieve themselves with great convenience. Thus, it is contemplated to provide a remote-controlled system to be incorporated into a bidet toilet seat system to be used for washing a body part and applying and delivery of a medicine.

Examples of such systems are disclosed in e.g. US 2009/313752 A1, US 2012/005817 A1, US 4 422 189 A, and US 2013/180041 A1.

### SUMMARY OF THE INVENTION

The present invention provides a remote-controlled delivery system according to claim 1. Further aspects and preferred embodiments are defined in the dependent claims.

The present disclosure generally provides a remote-controlled delivery system for delivering and applying medicines to a region of the body that may not otherwise be easily accessible (e.g., to the perianal region). A method of operating the remote-controlled delivery system is also provided. The remote-controlled delivery system is designed to enable the user to administer their medicine at an angle they control, while the user is in a relatively comfortable and efficacious position for the treatment. The system can present a safer, more hygienic, and more effective alternative method to self-administer perianal medicines than any option currently available. To this extent, the system can present a discreet, "hands-free" alternative to the current options, thereby substantially eliminating any discomfort, ineffectiveness, and/or embarrassment a user might otherwise experience.

A first aspect of the disclosure provides a remote-controlled system for the delivery of medicine, comprising: a nozzle delivery assembly having a remote nozzle applicator that delivers the medicine across a space to a localized region of a body of a user; a set of navigation control units that receive a user input; and a dynamic steering motor unit that adjusts at least one of an angle or a location of the medicine delivery apparatus based on the user input.

A second aspect of the disclosure provides a system for delivery of medicine to a perianal region of a user, comprising: a base unit (e.g., a bidet toilet seat washing system, a bidet medicine delivery seat, a toilet seat assembly, a bedpan, a hydraulic chair, a commode, a hospital bed, among others) adapted to receive the perianal region of the user; a nozzle delivery apparatus coupled to the base unit and having one or more remote nozzle applicator that delivers the medicine across a space to a localized region of the perianal region; a set of navigation control units that receive a user input; and a dynamic steering motor unit that adjusts at least one of an angle or a location of the medicine delivery apparatus based on the user input.

In one embodiment of this disclosure, a remote-controlled delivery system is provided and includes a toilet seat assembly, a nozzle delivery assembly inside the housing of the toilet seat assembly, a set of dynamic remote controls that receive a user input; and one or more dynamic steering motor units that adjusts at least one of an angle or a location of the nozzle delivery assembly based on the user input. In another embodiment, the remote-controlled delivery system further includes a medicine delivery assembly. In one aspect, one or more nozzles of the nozzle assembly are adapted to move back and forth, sideway, and/or rotatably in three-dimensional direction.

### BRIEF DESCRIPTION OF THE DRAWINGS

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope, for the invention may admit to other equally effective embodiments. The drawings are not necessarily to scale. The drawings are merely schematic representations, not intended to portray specific parameters of the invention.
Figure 1 is a perspective view of an example of a remote controlled delivery system according to embodiments of the invention.
Figure 2 is a side view of an example of a portion of a medicinal delivery system according to another embodiment of the invention.
Figure 3 is a perspective view of an example of a portion of a medicinal delivery system according to another embodiment of the invention.
Figure 4A illustrates examples of controls units that can be used for a remote controlled delivery system according to embodiments of the invention.
Figure 4B illustrates another example of controls units that can be used for a remote controlled delivery system according to embodiments of the invention.
Figure 4C illustrates another example of controls units that can be used for a remote controlled delivery system according to embodiments of the invention.
Figure 4D illustrates another example of controls units that can be used for a remote controlled delivery system according to embodiments of the invention.
Figure 5 shows one example of a medicinal storage and distribution assembly of the medicinal delivery system according to embodiments of the invention.
Figure 6 shows one example of a remote controlled medicinal delivery system in conjunction with a bidet wash seat system according to embodiments of the invention.

### DETAILED DESCRIPTION

The present disclosure generally includes a remote-controlled delivery system for delivering and applying medicines to a region of the body that may not otherwise be easily accessible (*e.g*., to the perianal region), a method of operating the remote-controlled delivery system. In one embodiment, the remote-controlled delivery system include a removable control unit coupled to a nozzle delivery assembly for the delivery of water and medication, *etc.,* to a surface area of a human subject.

In another embodiment, a remote-controlled delivery system is provided and includes a nozzle delivery assembly, a first motor being connected to the nozzle delivery assembly and capable of directing one or more extending and retracting movements of the nozzle delivery assembly, one or more second motors being connected to the nozzle delivery assembly and capable of directing one or more three-dimensional rotational movements of the nozzle delivery assembly, and one or more dynamic steering remote control units adapted to be communicating with both the first motor and the one or more second motors and directing one or more movements of the first motor and the one or more second motors.

In one embodiment, the nozzle delivery assembly includes a wash nozzle and a medicinal delivery nozzle. In one example, the wash nozzle may include a water jet head. In another example, the medicinal delivery nozzle includes a medicinal liquid inlet being connected to a medicinal storage assembly. In addition, the medicinal storage assembly includes one or more cartridges that store medicines to be applied, and a pressurized pump adapted to pump and deliver the medicines from the one or more cartridges to the medicinal liquid inlet. The medicinal delivery nozzle may further include a medicinal atomizer head adapted to modify a medicine to achieve a consistency that can be applied over a distance and deliver a stream of the medicine to an user. In one aspect, the medicinal delivery nozzle is adapted to deliver one or more medicines across a space to a localized region of a body of an user. One example of the localized region is a perianal region of the body of the user.

In another embodiment, the remote-controlled delivery system provided herein include one or more dynamic steering remote control units that receive a user input to direct the one or more movements of a first motor and one or more second motors and adjust at least one of an angle or a location of the delivery nozzle assembly based on the user input. In still another embodiment, the remote-controlled delivery system further includes a base unit coupled to the nozzle delivery assembly and adapted to receive a perianal region of an user. Examples of the base unit include, but are not limiting to, a bidet toilet seat washing system, a bidet medicine delivery seat, a toilet seat assembly, a bedpan, a hydraulic chair, a commode, a hospital bed, among others. In one example, the base unit of the remote-controlled delivery system is a toilet seat assembly, which contains a housing and a base seat.

In one aspect, the nozzle delivery assembly is positioned inside the housing of the toilet seat assembly. In another aspect, one or more nozzles of the nozzle delivery assembly of the remote-controlled delivery system are controlled by one or more dynamic steering remote control units and adapted to be moving in and out of the housing of the toilet seat assembly.

Additional embodiments of the disclosure provide one or more methods of operating and/or using a remote-controlled delivery system. The method includes controlling one or more movements of a nozzle delivery assembly by one or more dynamic steering remote control units. In one aspect, the one or more movements of a nozzle delivery assembly is controlled by one or more dynamic steering remote control units remotely via wireless means or long-wired means.

For example, the one or more dynamic steering remote control units are adapted to direct one or more extending and retracting movements of a nozzle delivery assembly by communicating the one or more dynamic steering remote control units with a first motor connected to the nozzle delivery assembly. In another example, the one or more dynamic steering remote control units are adapted to direct one or more three-dimensional rotational movements of the nozzle delivery assembly by communicating the one or more dynamic steering remote control units with one or more second motors.

In one aspect, the method includes jetting out a liquid solution from a water jet head of a wash nozzle of the nozzle delivery assembly. In another aspect, the method includes delivering one or more medicines from a medicinal storage assembly connected to a medicinal delivery nozzle of the nozzle delivery assembly, and spraying out the one or more medicines from a medicinal atomizer head of the medicinal delivery nozzle.

In still another aspect, the method further includes modifying the one or more medicines by the medicinal atomizer head so as to achieve a consistency that can be applied over a distance and deliver a stream of the one or more medicines to an user. In addition, the method further includes receiving an user input from the nozzle assembly by the one or more dynamic steering remote control units to direct one or more movements of the first motor and the one or more second motors and adjust at least one of an angle or a location of the delivery nozzle assembly based on the user input.

The nozzle assembly of the remote-controlled delivery system is easy-to-handle and easy to apply and spray water, fluids, solutions, suspensions, in combination of one or more medicament to a region (*e.g*., a skin area, *etc.*) of a person, and allow for easy operation and easy handling by an user controlling the dynamic remote control units manually. In one embodiment, a remote-controlled delivery system is incorporated into a base unit, such as a wash bidet system and is deigned to easily dispense water, liquid and medication from a wash, clean and dry bidet system to a surface area of a human subject.

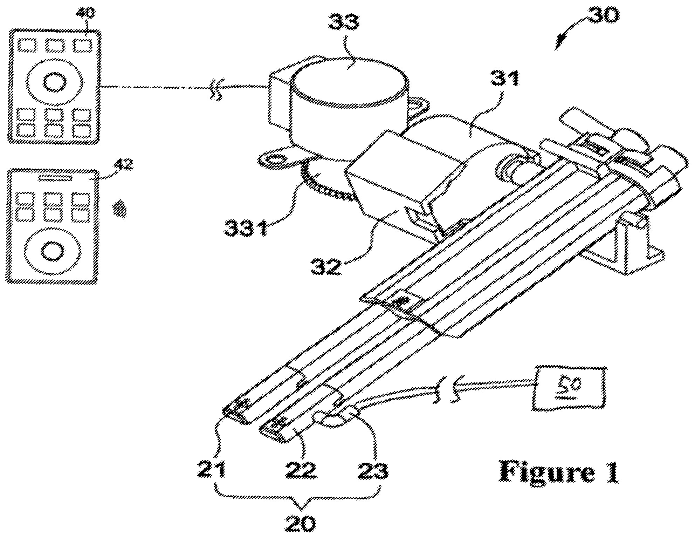

Figure 1 shows one example of a remote controlled delivery system according to embodiments of the disclosure. In one embodiment, the remote controlled delivery system includes a nozzle delivery assembly 20, a dynamic steering unit 30, and one or more dynamic steering remote control units 40, 42. The dynamic steering unit 30 include one or more motors 31, 32, 33 that are coupled to function coherently by one or more gears 331.

The dynamic steering remote control units 40, 42 are adapted to be communicating with the motors 31, 32, 33 and directing one or more movements of the motors 31, 32, 33. The motors 31, 32, 33 may be coupled together by the one or more gears 331 in order to coordinate and direct the movements of the nozzle delivery assembly 20. In addition, the remote controlled delivery system may further include a medicinal storage assembly 50.

As shown in Figure 1, the nozzle delivery assembly 20 includes a wash nozzle device 21 and a medicinal delivery nozzle device 22. In one aspect, the wash nozzle device 21 includes a water jet head for jetting water or any liquid stored within the wash nozzle device of the nozzle delivery assembly 20 to be sprayed out of the remote controlled delivery system. In one embodiment, the medicinal delivery nozzle device 22 includes a medicinal liquid inlet 23 being connected to the medicinal storage assembly 50.

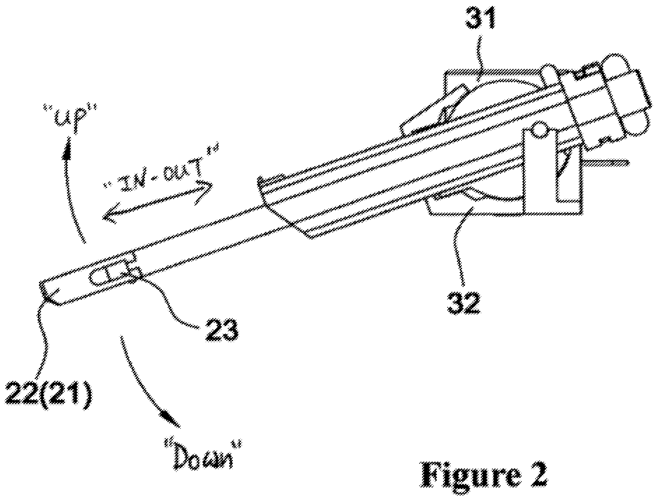

The dynamic steering unit 30 includes a motor 31 being connected to the nozzle delivery assembly 20 and capable of directing one or more extending and retracting movements of the nozzle delivery assembly 20 (also see Figure 2). The dynamic steering unit 30 includes one or more motors 32, 33 being connected to the nozzle delivery assembly 20 and capable of directing one or more three-dimensional rotational movements of the nozzle delivery assembly 20.

In Figure 1, the remote controlled delivery system 20 a remote applicator, such as the nozzle delivery assembly 20 that is adapted to couple the delivery of water and liquid in conjunction with one ore more medicines, medicate, medicinal, or other therapeutics across a space to a localized region (e.g., perianal region) of the body of a user by being spraying out of one ore more nozzle heads of the nozzle delivery assembly 20. As shown, the nozzle delivery assembly 20 includes a water jet head located at the front end of the wash nozzle device 21, a medicinal atomizer head located at the front end of the front tip end of the medicinal delivery device 22 near the medicinal liquid inlet 23.

In operation, the water jet head of the wash nozzle device 21 sprays out and applies a fluid to a region of the body of the user that is currently being targeted by a remote applicator, i.e., the nozzle delivery assembly 20. In this way, the currently targeted area can be indicated and it can be determined whether the currently target area is a localized region to which one or more medicine are to be applied by the medicinal deliver device 22. In one example, the water jet head of the wash nozzle device 21 uses water or a liquid other than the medicine to be used, the water jet head can be used (e.g., by being coupled to a sensor) to indicate whether the target area is the localized region to which the medicine is to be applied without the potential of wasting any medicine being applied on a region other that where it is intended. Further, the liquid applied by water jet head can be used to clean the region of the body (e.g., perianal region) prior to the application of the medicine by the medicinal delivery device 22.

As indicated above, aspects of the present disclosure provide a remote-controlled liquid and medicinal delivery system for delivering and applying water, liquid in combination with one or more medicines to a region of the body that may not otherwise be easily accessible (e.g., to the perianal region). To this extent, the remote controlled delivery system can present a discreet, "hands-free" alternative to the current options, thereby substantially eliminating any discomfort, ineffectiveness, and/or embarrassment a user might otherwise experience.

As indicated, the nozzle delivery system 20 also includes a medicinal atomizer head at the tip of the medicinal delivery device 22. The medicinal atomizer head delivers medicines to the target area. Medicines can be in the form of a liquid, a gel, a foam, a paste, or any other form that is now known or later developed that can be taken by a medicine. To this extent, the medicinal atomizer head of the medicinal delivery device 22 can modify the consistency of the medicine to achieve a consistency that can be applied over a distance (e.g., across a space). This modification can include, but is not limited to: aeration of a liquid medicine, aquafication of a gel or paste, and/or the like. In any case, the medicinal atomizer head is connected to the medicinal liquid inlet 23 for delivering one or more medicines from the medicinal storage assembly 50 to the medicinal atomizer head. To this extent, the water and medicinal atomizer can have the same target area on a surface region of a human user subject but function independently in terms of substances to be applied.

Figure 2 is a side view of an example of a portion of the remote controlled delivery system according to another embodiment of the present disclosure. As shown in Figure 2, the motors 31, 32, 33 may be coupled together in order to coordinate and direct the movements of the wash nozzle device 21 and the medicinal delivery nozzle device 22 of the nozzle delivery assembly 20. In one example, the motor 31 can be adapted to control the movement of the wash nozzle device 21 and the medicinal delivery nozzle device 22 to be able to move in a direction marked as "IN-OUT" to be extended and retracted in and out of the front end of the nozzle delivery assembly 20. In another example, the motors 32, 33 can be adapted to control the movement of the wash nozzle device 21 and the medicinal delivery nozzle device 22 to be able to move in a direction marked as "UP-DOWN" to be move up and down (e.g., in a Z-direction or a gravitational direction) around the front end of the nozzle delivery assembly 20, particularly when the nozzle heads of the wash nozzle device 21 and the medicinal delivery nozzle device 22 are being extended.

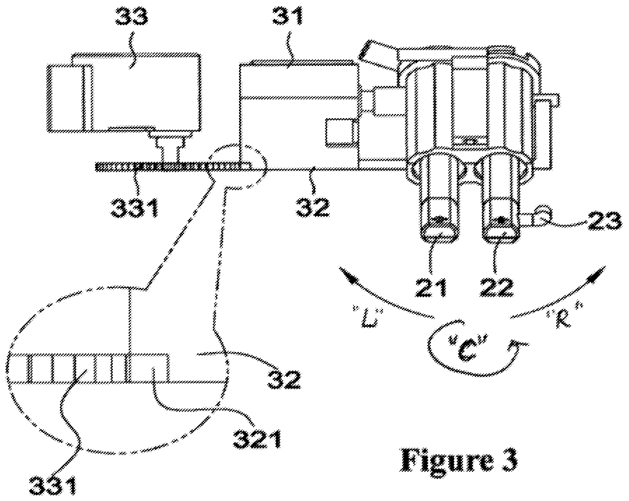

Figure 3 is a perspective view of an example of a portion of the remote controlled medicinal delivery system according to another embodiment of the present disclosure. The motors 31, 32, 33 may be coupled together by one or more gears 331, 332 in order to coordinate and direct the movements of the nozzle delivery assembly 20. In one example, the motors 31, 32, 33 can be adapted to control the movements of the wash nozzle device 21 and the medicinal delivery nozzle device 22 to be able to move in a direction marked as "LEFT-RIGHT" as well as "C" (*e.g*., circular, or rotational, in three-dimensional, *etc.*), particularly after the nozzle heads of the wash nozzle device 21 and the medicinal delivery nozzle device 22 are extended and retracted out at the front end of the nozzle delivery assembly 20.

Also shown in Figures 1-3, the dynamic steering unit 30 is controlled remotely by the one or more dynamic steering remote control units 40, 42 so as to function to direct the mechanical movements of the nozzle delivery assembly 20. The dynamic steering unit 30 includes one or more motors 31 that can be used to control angles (*e.g*., extending or retracting, raising up or lowering the angle of delivery respective to the body of the user) for the water and medicinal atomizer nozzle heads of the wash nozzle device 21 and the medicinal delivery nozzle device 22 systems. Further, one or more motors 33 with gears 331, 321, can be used to control the location of the nozzle heads of the nozzle delivery assembly 20. It should be understood that even though the dynamic steering unit 30 has been described in terms of motors and/or gears, any solution for altering the location and or angle of an apparatus should be envisioned.

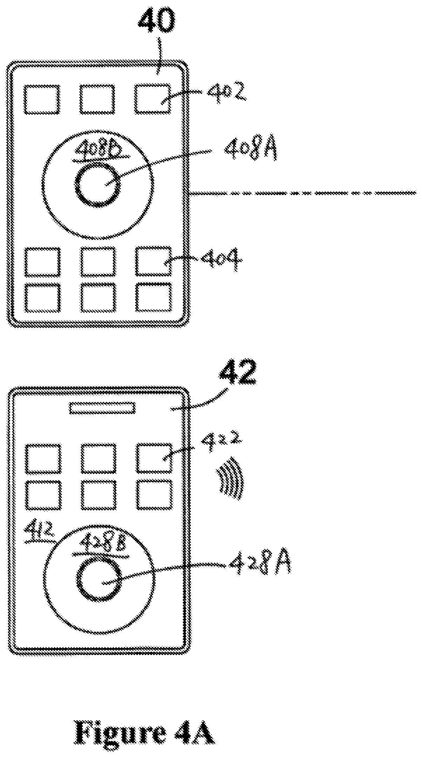

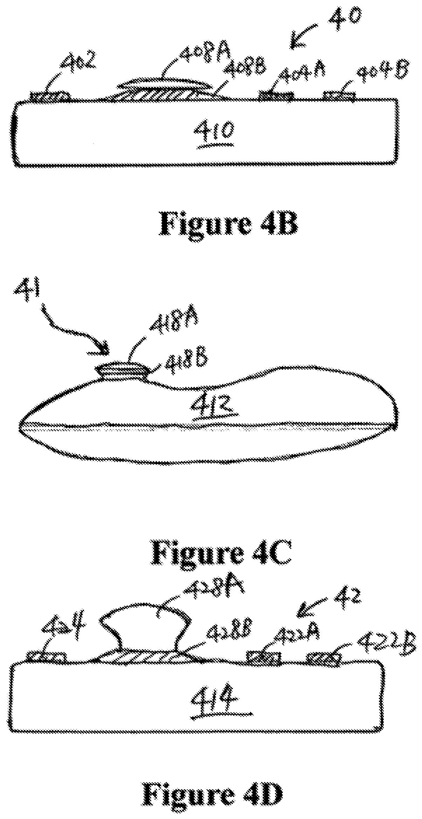

Figures 4A-4D illustrate examples of controls units, one or more dynamic steering remote control units 40, 41, 42 that can be used for a remote controlled delivery system according to embodiments of the present disclosure. In one embodiment, each of the dynamic steering remote control units 40, 41, 42, may include touch-sensitive panels controlled by an user's fingers. In other embodiments, the one or more dynamic steering remote control units 40, 41, 42 may include push buttons, a joystick, a trackball, a slider, a mouse, a wheel and/or any other solution now known or later developed.

In any case, navigation controls, such as the one or more dynamic steering remote control units 40, 41, 42 can receive an input from an user of the remote-controlled medicine delivery system and transmits the user input to the dynamic steering unit 30. Such transmission can occur via any wired or wireless solution now known or later developed. The dynamic steering unit 30 can receive the user input and adjust the angle and/or location of nozzle heads of the wash nozzle device 21 and the medicinal delivery device 22, based on the user input, and/or can begin the delivery of fluid from the water jet head or the medicinal atomizer in response to the user input. To this extent, navigation control commands for the one or more dynamic steering remote control units 40, 41, 42 can include controls for adjustment of the angle, adjustment of the locations of nozzles heads of the wash nozzle device 21 and the medicine delivery device 22 of the nozzle delivery assembly 20, indicating a start and/or stop to delivery of fluid from the water jet head, indicating a start and/or stop to delivery of medicine from the medicinal atomizer, and/or the like.

The one or more dynamic steering remote control units 40, 41, 42 may be further coupled to one or more power switches or control units such that various motors and electric circuits contained within the system can be turned on for powering up the system and operating water or liquid washing, and the delivery of one or more medicines, and/or other functions of the remote controlled medicinal delivery system. The control dynamic steering remote control units 40, 41, 42 can be positioned, in one example, on the top or the side of the system, and can be easily visible to a user for the user to grasp and manually controlled the system.

In one embodiment, at least one of the dynamic steering remote control units 40, 41, 42 is a remote control unit. In addition, one or more power indicators can be positioned on the dynamic steering remote control units 40, 41, 42 to indicate turning on of the electric power and proper functioning and control of the remote controlled medicinal delivery system.

In one example, as shown in Figure 4A, the dynamic steering remote control unit 40 include one or more control buttons 402, 404 for controlling various functions of the system. It may also include display screens, sensor screens, as well as touch sensing control pads that may include an user input sensor 408A and a control base 408A. The dynamic steering remote control unit 40 may be connected remotely in distance to the nozzle delivery assembly 20 and the motors 31, 32, 33 of the dynamic steering unit 30.

In Figure 4A, the dynamic steering remote control unit 42 includes a power indicator, one or more control buttons 422 for controlling various functions of the system. The dynamic steering remote control unit 42 may additional includes one or more user input sensor 428A and control base 428A can be connected remotely in distance to the nozzle delivery assembly 20 and the motors 31, 32, 33 of the dynamic steering unit 30 by wireless communication, as exemplarily shown in Figure 4A.

Figures 4B-4D illustrate side views of the dynamic steering remote control units 40, 41, 42, respectively. The dynamic steering remote control units 40, 41, 42, may include base bodies 410, 412, 414, respectively, for storing electric circuits and other necessary electronic parts for the functioning of the dynamic steering remote control units 40, 41, 42. In operation, an user can direct the control of the remote control medicinal delivery system by directly touches one or more control buttons 402, 404A, 404B, 422A, 422B, 424, *etc.,* positioned on top of the base bodies 410, 412, 414. Each button may be adapted to direct to one or more functioning commands (e.g., washing, moving up and down, moving left and right, extending and retracting, medicine delivery, *etc.*) for the functioning of various parts and components of the remote controlled medicinal delivery system to be carried out by the dynamic steering remote control units 40, 41, 42.

Alternatively, an user can direct the control of the remote control medicinal delivery system by directly touching one or more sensor pads, sensor mouse-like or joystick-like sensor controls. The sensor pads or joystick-like sensor controls may include one or more user control bases 408B, 418B, and 428B, and one or more input sensors 408A, 418A, 428A, *etc.,* positioned on top of the base bodies 410, 412, 414. Each input sensor 408A, 418A, 428A, *etc.,* may be adapted to direct to all of the functioning commands (e.g., washing, moving up and down, moving left and right, extending and retracting, medicine delivery, etc.) for the functioning of various parts and components of the remote controlled medicinal delivery system to be carried out by the dynamic steering remote control units 40, 41, 42.

In one aspect, the remote controlled medicinal delivery system is connected to a power cord via a power connector. The power cord is adapted to be connected to an electric outlet and provides electric power to power up the system. In another aspect, the wash and dry system is connected to a battery power pack in order to conveniently power up the wash and dry system without the need to find an electric outlet.

In operation, once the electric power is turned on and the motors within the system are adapted to deliver all desirable liquids (*e.g*., water and liquid from a water hose assembly via a water inlet, a medication liquid solution from the medicine storage assembly 50, and combinations thereof) from one or more channels therein to the nozzle delivery assembly.

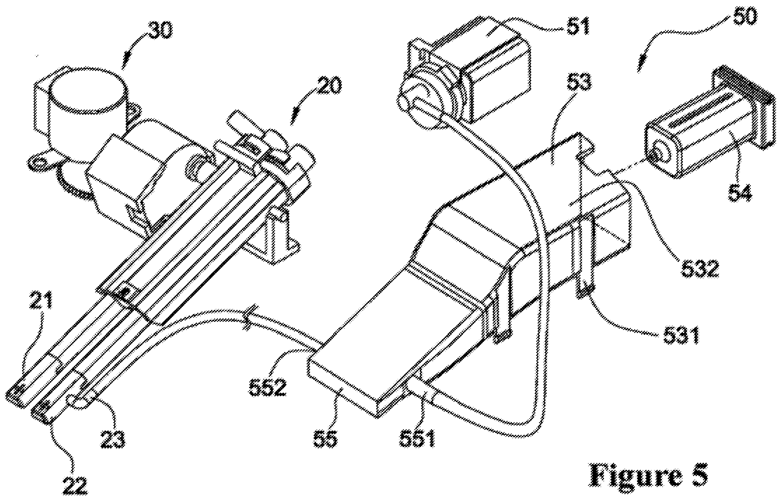

Figure 5 shows a medicinal storage and delivery assembly of the remote controlled medicinal delivery system according to embodiments of the present disclosure. One example of the medicinal storage and delivery assembly is a medicinal storage assembly 50 which includes one or more liquid cartridges 54 that hold one or more kinds of medicines, a pump 51, that is connected to the liquid cartridge 54 via one or more channels 551, 552 to be used to pressurize medicines for delivering the medicine from the one or more liquid cartridges 54 to the medicinal atomizer nozzle head of the medicinal delivery nozzle 22 through the medicinal liquid inlet 23. The pump 51 can include any solutions for moving a liquid or other viscous substance that is now known or later developed and can be powered by any power source that is now known or later developed. In general, one or more medicines are stored in the one or more cartridges 54 positioned within a medicinal storage housing 53. The medicinal storage housing 53 may include a body 532 and one or more fasteners 531. The medicinal storage housing 53 may be connected to a delivery housing 55 for coupling to the pump 51 via the channels 551, 552.

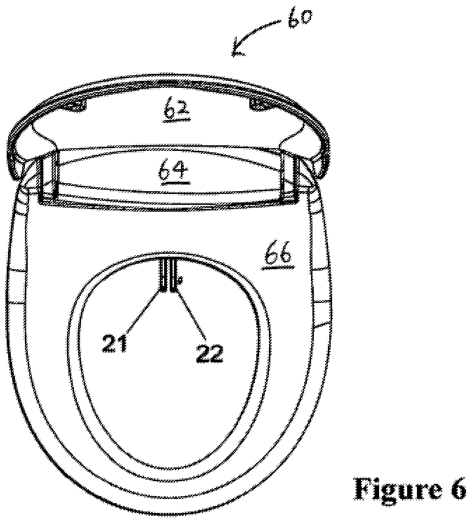

Figure 6 shows one example of a remote controlled medicinal delivery system in conjunction with a base unit, *e.g*., a bidet wash seat system, according to embodiments of the present disclosure. One example of a remote controlled medicinal delivery system used in conjunction with a base unit. One example is a wash toilet with a bidet seat device as shown in Figure 6 according to embodiments of the present disclosure. In an embodiment, the integrated structure includes a toilet seat assembly 60 that contains a seat cover 62, a base housing 62, and a seat body 64.

As shown in Figure 6, the nozzle heads of the wash nozzle device 21 and the medicinal delivery nozzle device 22 (shown fully extended), being stored within the base housing 64, can be seen emerging from a bottom side of the seat body 64. The wash nozzle device 21 and the medicinal delivery nozzle device 22 of the nozzle delivery assembly 20 are sued to deliver a jet of water and a spray of medicine, respectively, to the user.

Although shown in Figure 6 as being used in conjunction with a wash toilet seat, it should be understood that the remote control medicinal delivery system as mentioned herein is contemplated to be adapted to a variety of devices intrinsic to Activities of Daily Living (ADL), including, but not limited to, a hydraulic chair, a wash toilet seat, a commode, a specially-modified hospital bed and/or the like. Further, those skilled in the art should appreciate that portable devices such as a bedpan or other portable apparatuses that are now known or later developed are also envisioned. In each case, the result of the incorporation of a remote control medicinal delivery system into those ADL devices is that perianal medicine will now be capable of being administered in a more effective and discreet manner across the whole spectrum of users' postural limitations around which any given ADL device is designed.

While the foregoing is directed to embodiments of the present disclosure, other and further embodiments may be devised without departing from the basic scope thereof, the scope of the invention being determined by the claims that follow.

## Claims

1. A remote-controlled delivery system, comprising:
a nozzle delivery assembly including a wash nozzle (21) and a medicinal delivery nozzle (22);
a first motor (31) being connected to the nozzle delivery assembly (20) and capable of directing one or more extending and retracting movements of the nozzle delivery assembly (20);
one or more second motors (32, 33) being connected to the nozzle delivery assembly (20) and capable of directing one or more three-dimensional rotational movements of the nozzle delivery assembly (20); and
one or more dynamic steering remote control units (40, 41, 42) adapted to be communicating with both the first motor (31) and the one or more second motors (32, 33) and directing one or more movements of the first motor (31) and the one or more second motors (32, 33);
wherein the first motor (31) is configured to move one or both of the wash nozzle (21) and the medicinal delivery nozzle (22) between a retracted position and an extended position in response to the one or more dynamic steering remote control units (40, 41, 42) receiving a user input;
wherein one of the one or more second motors (32, 33) is configured to rotate one or both of the wash nozzle (21) and the medicinal delivery nozzle (22) in response to the one or more dynamic steering remote control units (40, 41, 42) receiving the user input.

2. The remote-controlled delivery system of claim 1, wherein the wash nozzle (21) further comprises a water jet head.

3. The remote-controlled delivery system of claim 2, wherein the medicinal delivery nozzle (22) further comprises a medicinal liquid inlet (23) being connected to a medicinal storage assembly (50);
preferably wherein the medicinal storage assembly (50) further comprises:
one or more cartridges (54) that stores medicines to be applied, and a pressurized pump (51) adapted to pump and deliver the medicines from the one or more cartridges (54) to the medicinal liquid inlet (23).

4. The remote-controlled delivery system of claim 2, wherein the medicinal delivery nozzle (22) further comprises a medicinal atomizer head adapted to modify a medicine to achieve a consistency that can be applied over a distance and deliver a stream of the medicine to an user.

5. The remote-controlled delivery system of claim 1, wherein the one or more dynamic steering remote control units (30) receive a user input to direct the one or more movements of the first motor (31) and the one or more second motors (32, 33) and adjust at least one of an angle or a location of the delivery nozzle assembly based on the userinput.

6. The remote-controlled delivery system of claim 1, further comprising a base unit coupled to the nozzle delivery assembly (20) and adapted to receive a perianal region of a user.

7. The remote-controlled delivery system of claim 6, wherein the base unit comprises a toilet seat assembly (60) comprising a housing (62) and a base seat (64).

8. The remote-controlled delivery system of claim 7, wherein the nozzle delivery assembly (20) is positioned inside the housing (62) of the toilet seat assembly (60).

9. The remote-controlled delivery system of claim 8, wherein one or more nozzles of the nozzle delivery assembly (20) are controlled by the one or more dynamic steering remote control units (30) and adapted to be moving in and out of the housing (62).

10. The remote-controlled delivery system of claim 6, wherein the base unit comprises a bedpan, or a hydraulic chair, or a commode, or a hospital bed.

## Patentansprüche

1. Ferngesteuertes Abgabesystem, Folgendes umfassend:
eine Düsenabgabeanordnung, die eine Spüldüse (21) und eine medizinische Abgabedüse (22) beinhaltet;
einen ersten Motor (31), der mit der Düsenabgabeanordnung (20) verbunden ist und der eine oder mehrere Ausfahr- und Einfahrbewegungen der Düsenabgabeanordnung (20) lenken kann;
**einen oder mehrere zweite Motoren (32, 33), die mit der** Düsenabgabeanordnung (20) verbunden sind und eine oder mehrere dreidimensionale Drehbewegungen der Düsenabgabeanordnung (20) lenken können; und
eine oder mehrere dynamische Lenkfernsteuerungseinheiten (40, 41, 42), die so ausgelegt sind, dass sie sowohl mit dem ersten Motor (31) als auch dem einen oder den mehreren zweiten Motoren (32, 33) in Verbindung stehen und eine oder mehrere Bewegungen des ersten Motors (31) und des einen oder der mehreren zweiten Motoren (32, 33) lenken;
wobei der erste Motor (31) so ausgebildet ist, dass er eines oder beides aus der Spüldüse (21) und der medizinischen Abgabedüse (22) zwischen einer eingefahrenen Position und einer ausgefahrenen Position als Reaktion darauf **bewegt, dass die eine oder die mehreren dynamischen** Lenkfernsteuerungseinheiten (40, 41, 42) eine Benutzereingabe empfangen;
wobei einer des einen oder der mehreren zweiten Motoren (32, 33) so ausgebildet ist, dass er eine oder beide aus der Spüldüse (21) und der medizinischen Abgabedüse (22) als Reaktion auf die eine oder die mehreren dynamischen Lenkfernsteuerungseinheiten (40, 41, 42), die die Benutzereingabe empfangen, dreht.

2. Ferngesteuertes Abgabesystem nach Anspruch 1, wobei die Spüldüse (21) ferner einen Wasserstrahlkopf umfasst.

3. Ferngesteuertes Abgabesystem nach Anspruch 2, wobei die medizinische Abgabedüse (22) ferner einen Einlass für eine medizinische Flüssigkeit (23) umfasst, der mit einer medizinischen Lageranordnung (50) verbunden ist;
wobei die medizinische Lageranordnung (50) bevorzugt ferner Folgendes umfasst:
eine oder mehrere Kartuschen (54), in denen die aufzutragenden Medikamente gelagert werden, und eine Druckpumpe (51), die so ausgelegt ist, dass sie die Medikamente von der einen oder den mehreren Kartuschen (54) zum Einlass für eine medizinische Flüssigkeit (23) pumpt und abgibt.

4. Ferngesteuertes Abgabesystem nach Anspruch 2, wobei die medizinische Abgabedüse (22) ferner einen medizinischen Zerstäuberkopf umfasst, der so ausgelegt ist, dass er ein Medikament so modifiziert, dass eine Konsistenz erreicht wird, die über eine Strecke aufgebracht werden kann, und einen Strahl des Medikaments an einen Benutzer abgibt.

5. Ferngesteuertes Abgabesystem nach Anspruch 1, wobei die eine oder die mehreren dynamischen Lenkfernsteuerungseinheiten (30) eine Benutzereingabe empfangen, um die eine oder die mehreren Bewegungen des ersten Motors (31) und des einen oder der mehreren zweiten Motoren (32, 33) zu lenken und zumindest einen Winkel oder eine Position der Abgabedüsenanordnung basierend auf der Benutzereingabe einzustellen.

6. Ferngesteuertes Abgabesystem nach Anspruch 1, das ferner eine Basiseinheit umfasst, die mit der Düsenabgabeanordnung (20) gekoppelt ist und so ausgelegt ist, dass sie eine Perianalregion eines Benutzers aufnimmt.

7. Ferngesteuertes Abgabesystem nach Anspruch 6, wobei die Basiseinheit eine Toilettensitzanordnung (60) mit einem Gehäuse (62) und einem Basissitz (64) umfasst.

8. **Ferngesteuertes Abgabesystem nach Anspruch 7, wobei die** Düsenabgabeanordnung (20) in dem Gehäuse (62) der Toilettensitzanordnung (60) angeordnet ist.

9. Ferngesteuertes Abgabesystem nach Anspruch 8, wobei eine oder mehrere Düsen der Düsenabgabeanordnung (20) durch die eine oder mehreren dynamischen Lenkfernsteuerungseinheiten (30) gesteuert werden und so ausgelegt sind, dass sie sich in und aus dem Gehäuse (62) heraus bewegen.

10. Ferngesteuertes Abgabesystem nach Anspruch 6, wobei die Basiseinheit eine Bettpfanne oder einen hydraulischen Stuhl oder einen Toilettenstuhl oder ein Krankenhausbett umfasst.

## Revendications

1. Système de distribution commandé à distance, comprenant :
un ensemble de distribution à buse comprenant une buse de lavage (21) et une buse de distribution de médicament (22) ;
un premier moteur (31) étant connecté à l'ensemble de distribution à buse (20) et capable de diriger un ou plusieurs mouvements d'extension et de rétraction de l'ensemble de distribution à buse (20) ;
un ou plusieurs deuxièmes moteurs (32, 33) étant connectés à l'ensemble de distribution à buse (20) et capables de diriger un ou plusieurs mouvements de rotation tridimensionnels de l'ensemble de distribution à buse (20) ; et
une ou plusieurs unités de commande à distance de direction dynamique (40, 41, 42) adaptées pour communiquer à la fois avec le premier moteur (31) et le ou les deuxièmes moteurs (32, 33) et pour diriger un ou plusieurs mouvements du premier moteur (31) et le ou les deuxièmes moteurs (32, 33) ;
dans lequel le premier moteur (31) est configuré pour déplacer l'une ou les deux buses de lavage (21) et la buse de distribution de médicament (22) entre une position rétractée et une position étendue en réponse à la ou aux unités de commande à distance de direction dynamique (40, 41, 42) recevant une entrée d'utilisateur ;
dans lequel le ou les deuxièmes moteurs (32, 33) sont configurés pour faire tourner l'une ou les deux buses de lavage (21) et la buse de distribution de médicament (22) en réponse à l'une ou aux plusieurs unités de commande à distance de direction dynamique (40, 41, 42) recevant l'entrée d'utilisateur.

2. Système de distribution commandé à distance selon la revendication 1, dans lequel la buse de lavage (21) comprend en outre une tête à jet d'eau.

3. Système de distribution commandé à distance selon la revendication 2, dans lequel la buse de distribution de médicament (22) comprend en outre une entrée de liquide médicamenteux (23) reliée à un ensemble de stockage de médicament (50) ;
de préférence dans lequel l'ensemble de stockage de médicaments (50) comprend en outre :
une ou plusieurs cartouches (54) qui stockent des médicaments à appliquer, et une pompe pressurisée (51) adaptée pour pomper et distribuer les médicaments depuis une ou plusieurs cartouches (54) vers l'entrée de liquide médicamenteux (23).

4. Système de distribution commandé à distance selon la revendication 2, dans lequel la buse de distribution de médicament (22) comprend en outre une tête de pulvérisation de médicament adaptée pour modifier un médicament pour obtenir une consistance qui peut être appliquée sur une distance et délivrer un flux du médicament à un utilisateur.

5. Système de distribution commandé à distance selon la revendication 1, dans lequel les une ou plusieurs unités de commande à distance de direction dynamique (30) reçoivent une entrée d'utilisateur pour diriger les un ou plusieurs mouvements du premier moteur (31) et les un ou plusieurs deuxièmes moteurs (32, 33) et pour régler au moins un angle ou un emplacement de l'ensemble de distribution à buse sur la base de l'entrée d'utilisateur.

6. Système de distribution commandé à distance selon la revendication 1, comprenant en outre une unité de base couplée à l'ensemble de distribution à buse (20) et adaptée pour recevoir une région périanale d'un utilisateur.

7. Système de distribution commandé à distance selon la revendication 6, dans lequel l'unité de base comprend un ensemble de siège de toilette (60) comprenant un logement (62) et un siège de base (64).

8. Système de distribution commandé à distance selon la revendication 7, dans lequel l'ensemble de distribution à buse (20) est positionné à l'intérieur du logement (62) de l'ensemble de siège de toilette (60).

9. Système de distribution commandé à distance selon la revendication 8, dans lequel une ou plusieurs buses de l'ensemble de distribution à buses (20) sont commandées par une ou plusieurs unités de commande à distance de direction dynamique (30) et adaptées pour être déplacées à l'intérieur et à l'extérieur du logement (62).

10. Système de distribution commandé à distance selon la revendication 6, dans lequel l'unité de base comprend un bassin de lit, ou une chaise hydraulique, ou une chaise percée, ou un lit d'hôpital.
